(19) Europäisches Patentamt
European Patent Office
Office européen des brevets

(11) Publication number: **0 304 125 B1**

(12) **EUROPEAN PATENT SPECIFICATION**

(45) Date of publication of patent specification: **23.03.94**

(51) Int. Cl.5: **C08G 59/62**, C07C 39/367, C08G 59/06, C07C 37/20, C07C 29/32

(21) Application number: **88201727.0**

(22) Date of filing: **11.08.88**

(54) **Heat-hardenable compositions based on polyglycidyl ethers and polyhydric phenols, halogenated tetraphenols, polyglycidyl ethers and process for preparing tetraphenols.**

(30) Priority: **19.08.87 GB 8719616**

(43) Date of publication of application: **22.02.89 Bulletin 89/08**

(45) Publication of the grant of the patent: **23.03.94 Bulletin 94/12**

(84) Designated Contracting States: **BE DE ES FR GB IT NL SE**

(56) References cited:
**EP-A- 0 186 893**
**EP-A- 0 230 961**
**FR-A- 1 523 850**
**GB-A- 859 456**
**US-A- 2 806 016**
**US-A- 2 857 362**
**US-A- 3 218 370**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Raudenbusch, Werner Theodor**
**Badhuisweg 3**
**NL-1031 CM Amsterdam(NL)**
Inventor: **Verburg, Antoon**
**Badhuisweg 3**
**NL-1031 CM Amsterdam(NL)**
Inventor: **de Kruif, Jan Hendrik**
**Badhuisweg 3**
**NL-1031 CM Amsterdam(NL)**
Inventor: **van Acker, Eduard M. A. A. J.**
**Badhuisweg 3**
**NL-1031 CM Amsterdam(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to heat-hardenable compositions comprising mixtures of polyglycidyl ethers and polyphenolic crosslinking agents and to their use in the production of laminated products.

Laminates generally comprise a reinforcing material of which different layers are bonded together via a hardened thermosetting binder composition. A conventional method for making such laminates is to impregnate a glass fibre fabric with an epoxy binder system followed by compression moulding one or more layers of the impregnated fabric. A primary requirement for such binder compositions is that they have good bonding properties. A further requirement especially for those laminates which are used in the production of printed circuit boards, is that the crosslinked binder matrix should have a good thermal stability. It has long been established that polyfunctional epoxy resin based binder systems are well suited to be used in the production of laminates. Examples of such epoxy resin based binders are described in US A 3,218,370 and comprise a combination of 1) a polyglycidyl ether having an epoxy functionality of at least three, derived from a polyphenol having from three to four phenylol groups in its molecule which are connected via an aliphatic hydrocarbon group, and 2) a stoichiometric amount of a phenolformaldehyde condensate, having a phenolic hydroxyl functionality of at least three. Those known laminates will meet most of the heat stability requirements for conventional printed circuit boards. However, with the advent of more sophisticated printed circuit boards which are based on thinner metal conductive strips and which have a larger number of such strips per unit surface area, the thermal stability of the binder matrices from the prior art may not always be sufficient. Hence, there is a growing demand for heat-hardenable binder compositions for use in the production of laminates having increased thermal stability.

A criterion frequently employed when selecting novel heat-hardenable binder systems, e.g for use in laminates, is the glass transition temperature (Tg) of the hardened binder system, as this gives an indication of the stability of the matrix to withstand processing, e.g soldering at 260 °C. The Tg is also taken as a criterion for the dimensional stability in the processing temperature range. The problem underlying the present invention is to provide heat-hardenable binder systems for use in laminate production which have improved thermal stability or alternatively a higher Tg than existing systems.

It has now been found that this problem can be solved by providing heat-hardenable compositions comprising mixtures of

a) at least a polyglycidyl ether of a tetraphenol having on average at least three epoxy groups per molecule, and

b) at least a tetraphenolic crosslinking agent

in a weight ratio sufficient to provide an epoxy group to phenolic hydroxyl group equivalent ratio in the range of from 0.5 to 1.5, and wherein the tetraphenol from which the polyglycidyl ether is derived and the tetraphenolic crosslinking agent are represented by the general formula

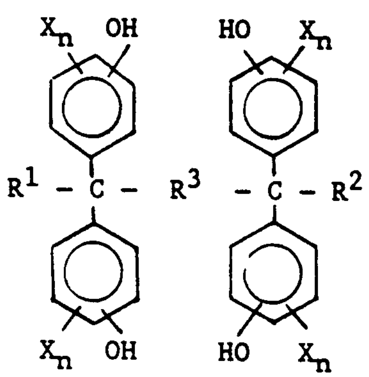

(I)

wherein $R^1$ and $R^2$ are H or a $C_{1-3}$ alkyl group, $R^3$ is a linear $C_{2-6}$ alkylene group which may carry one or more alkyl substituents, each n individually is 0,1 or 2 and X is Cl or Br. Preferred heat-hardenable compositions are those wherein in general formula I $R^1$ and $R^2$ are H. Especially preferred are such compositions wherein in general formula I $R^3$ is a linear propylene group.

The tetraphenols of general formula I from which the polyglycidyl ethers are derived and on which the tetraphenolic crosslinking agents are based and which may be employed in the heat-hardenable compositions of the present invention include:

1,1,4,4-tetrakis(hydroxyphenyl) butane,.

1,1,5,5 tetrakis(hydroxyphenyl) pentane,

1,1,5,5-tetrakis(hydroxyphenyl)-3-methyl pentane,
1,1,5,5-tetrakis(3-bromo-4-hydroxyphenyl) pentane,
1-(3-chloro-4-hydroxyphenyl)-1,5,5 tris(4-hydroxyphenyl) pentane,
1-(3-bromo-2-hydroxyphenyl)-1,5,5-tris(3-bromo-4-hydroxyphenyl) pentane,
1,1,5,5-tetrakis(3,5-dibromo-4-hydroxyphenyl) pentane,
2,2,9,9-tetrakis(hydroxyphenyl) decane.

The heat-hardenable compositions of the present invention are based on well defined compounds which can conveniently be prepared on a commercial scale. Furthermore the hydrocarbon group connecting the phenylol groups of the tetraphenol of general formula I, affords good solubility of the individual compounds in combination with a high glass transition temperature and good mechanical performance properties of the hardened compositions. Finally those compositions wherein in general formula I n / O have improved flame retardancy upon hardening.

With the tetraphenols of general formula I the phenolic hydroxyl groups will generally occupy the para or an ortho position with respect to the hydrocarbon substituent group. Preferred however are tetraphenols of general formula I wherein the phenolic hydroxyl groups predominantly occupy the para position.

The tetraphenols of general formula I, wherein $R^1$ and $R^2$ are H and each n is O, can readily be obtained by condensing a linear $C_{4-8}$ dialdehyde with phenol following the method as described e.g. in US-A 2,806,016. A disadvantage of said method is that it requires a reaction time of several days. A preferred method for the preparation of such tetraphenols from the same reactants comprises reacting phenol and a linear $C_{4-8}$ dialdehyde in the presence of hydrochloric acid, characterized in that the molar ratio of phenol and dialdehyde is at least 10:1 and that the hydrochloric acid is present in a concentration of at least 0.5 % mol calculated on phenol intake, and the dialdehyde is gradually added as an aqueous solution to the mixture of molten phenol and hydrochloric acid at such a rate that the temperature in the reactor does not rise above 65 °C. and wherein subsequently the volatiles are removed by distillation at elevated temperature and subatmospheric pressure. Upon completion of the dialdehyde addition it is beneficial to allow for a relatively short post-reaction period, e.g. 30 minutes at approximately 50 °C. By this method the envisaged tetraphenol may be obtained in yields which are well above 85 % m.

The tetraphenols of general formula I wherein $R^1$ and $R^2$ are a $C_{1-3}$ alkyl group, can be prepared via a process wherein phenol is reacted with an appropriate linear $C_{8-12}$ diketone in the presence of a hydrochloric acid/mercaptan catalyst system.

The chlorine or bromine containing tetraphenols of general formula I can be obtained by reaction of the corresponding halogen-free tetraphenol with chlorine or bromine respectively, via methods well known in the art. Said halogen groups will usually be substituted on the ortho positions of the phenyl rings with respect to the phenolic hydroxyl group, although configurations wherein such a halogen group occupies the para position with respect to the phenolic hydroxyl group may also occur when said phenolic hydroxyl group occupies an ortho position with respect to the hydrocarbon substituent. Preferred however are such halogenated tetraphenols wherein the groups X predominantly occupy an ortho position with respect to the phenolic hydroxyl group. The value for n in general formula I will be determined by the molar excess of chlorine or bromine employed in the halogenation process step.

The tetraphenols of general formula I wherein $R^1$ and $R^2$ are H or a $C_{1-3}$ alkyl group, $R^3$ is a $C_{2-3}$ linear alkylene group, n in at least one of the four phenylol entities is 1 or 2 and X is Cl or Br, are novel compounds.

The polyglycidyl ethers which may be employed in the heat-hardenable compositions of the present invention can be prepared according to the method as described in US-A 3.452.116. It is preferred however, to prepare these polyglycidyl ethers based on the tetraphenols of general formula I via a process wherein the tetraphenol is dissolved in a large molar excess, with respect to the intake of phenol hydroxyl, of an epihalohydrin preferably epichlorohydrin, and 2-propanol. The solution is heated to a temperature in the range of from 40 to 50 °C whereupon a 15 to 50 % m aqueous sodium hydroxide solution is added over a period of about 30 minutes, in an amount which is sufficient to provide a slight molar excess of sodium hydroxide with respect to the phenol hydroxyl intake, and in which period the temperature is allowed to increase to a temperature in the range of from 60 to 70 °C. After a short post reaction time (30 -60 min) at that temperature the organic phase is separated off, washed and dissolved in a suitable, partly water-miscible solvent such as methyl isobutyl ketone and further treated with a small amount of approximately a 4-6 % m aqueous sodium hydroxide solution at the reflux temperature of the water and methyl isobutyl ketone. After a further washing step and removal of the volatiles in vacuo the polyglycidyl ethers are obtained as a pale yellow solid material. Preferred such polyglycidyl ethers have on average 3.8 to 4.0 epoxy groups per molecule.

Polyglycidyl ethers having at least three epoxy groups per molecule and which are derived from the tetraphenols of general formula I wherein $R^1$ and $R^2$ are H or a $C_{1-3}$ alkyl group, $R^3$ is a $C_{2-3}$ linear alkylene group, n in at least one of the four phenylol entities is 1 or 2 and X is Cl or Br, are novel compounds.

The polyglycidyl ethers which may be employed in the heat-hardenable compositions of the present invention may be chosen from single polyglycidyl ethers as herein before defined or from mixtures of such polyglycidyl ethers, e.g mixtures of polyglycidyl ethers having a different degree of halogenation or different halogen atoms.

The tetraphenol crosslinking agent which may be used in the heat-hardenable compositions of the present invention may be a single tetraphenol as hereinbefore defined or a mixture of such tetraphenols e.g mixtures of tetraphenols having a different degree of halogenation or different halogen atoms.

It is preferred that the polyglycidyl ether(s) and the tetraphenol crosslinking agent(s) are present in the heat-hardenable composition of the present invention in a weight ratio which is sufficient to provide an epoxy group to phenolic hydroxyl group equivalent ratio in the range of from 0.8 to 1.2.

Although the heat-hardenable compositions may be hardened as such by heating at an elevated temperature it has been found beneficial to incorporate small amounts of a catalyst which promotes the hardening reaction between the polyglycidyl ether and the tetraphenol crosslinking agent. Suitable catalysts may be selected from the group of catalysts comprising tertiary amines, quarternary ammonium salts, quarternary phosphonium salts, tertiary sulfonium salts, imidazoles and Lewis acids. The actual amount of catalyst to be employed will be determined by the nature of the catalyst as well as the hardening conditions and will generally be in the range of from 0.001 - 1.0 % m on total solids of the composition.

The heat-hardenable compositions of the present invention may conveniently be hardened at a temperature in the range of from 150 to 250 °C and preferably from 180 to 220 °C.

When a heat hardenable composition of the present invention is employed for the preparation of laminates it is dissolved in a solvent to facilitate the impregnation of the reinforcement, such a glass fibre fabric. The nature of the solvent is not critical provided said solvent is inert. It is preferred however, to employ solvents with a high solvent power and a low boiling point so that only relatively small amounts of solvents have to be employed in preparing the impregnation solution. This also enables fast solvent evaporation from the impregnated fabric. Suitable solvents include organic esters, ketones, mixtures of alcohols and selected hydrocarbons. Ketones are preferred.

If desired, pigments, fillers, surface active agents, stabilisers, antioxidants and other auxiliary compounds may also be included in the heat-hardenable composition of the present invention.

The invention will be further illustrated by the following examples.

## Example I

### Preparation of tetraphenylol pentane

Phenol (1128 g, 12 mol) was melted in a nitrogen-flushed glass reactor at 47 °C which was followed by the addition of concentrated (37 % m) hydrochloric acid (11.8g, 4.4 g HCl). Glutaraldehyde (160 g of a 50 % m aqueous solution, 0.8 mol) was added with stirring over a period of 0.5 h during which the temperature in the reactor was allowed to increase to a maximum of 55 °C. After a post-reaction period of 15 min. at 50 °C the volatiles were removed in vacuo (160 °C/17 mbar) yielding 330 g of tetraphenylol pentane (94 % yield). The end product melted between 110 and 120 °C, had a phenolic hydroxyl content of 9100 mmol/kg and was found to contain less than 0.5 % m phenol.

## Example II

### Preparation of brominated tetraphenylol pentane

Tetraphenylol pentane (220 g, 0.5 mol) as prepared in example I was dissolved in a blend of dichloro methane (450 ml) and methanol (150 ml). The solution was cooled to -35 °C and maintained at that temperature while bromine (320 g, 2 mol $Br_2$) was added with stirring over a period of two hours. Subsequently the temperature was raised to 20 °C and the solution was washed with an aqueous sodium bicarbonate solution until neutral which was followed by evaporation of the volatiles in vacuo (120 °C, 2 mbar). The brittle brownish product (375 g) melted at a temperature between 70 - 80 °C and had a phenolic hydroxyl of 5300 mmol/kg and a bromine content of 42.3 % m.

Example III

Preparation of polyglycidyl ether from brominated tetraphenylol pentane

Brominated tetraphenylol pentane (302 g, 0.4 mol) as prepared in example II was dissolved in a blend of epichlorohydrin (740 g, 8 mol) and 2-propanol (336 g). The solution was heated to 45 °C whereupon an aqueous solution of sodium hydroxide (68,8 g NaOH in 73 g water) was added over a period of 30 minutes with stirring in which time the temperature was raised to 70 °C. After a post-reaction period of 15 minutes the organic phase was separated from the brine and washed with a 3 % m $NaH_2PO_4$ solution (250 ml) and water (250 ml). After removal of the volatiles at 100 °C and 20 mbar the residue (400 g) was dissolved in methyl isobutyl ketone (600 g) and treated with a 5 % m aqueous NaOH solution (60 g) for 1.5 hours at 90 °C. After a further washing with a 3 % m aqueous $NaH_2PO_4$ solution (250 ml) and water (250 ml) the solvent was stripped off in vacuo (120 °C, 2 mbar). The final product was a pale yellow slightly tacky epoxy resin (385 g) having an epoxy group concentration: 3610 mmol/kg, a saponifiable chlorine content: 65 ppm and a bromine content: 32.7 % m.

Example IV

Preparation of polyglycidyl ether from tetraphenylol pentane

Tetraphenylol pentane (220 g, 0.5 mol) as prepared according to the method of Example I was dissolved in a blend of epichlorohydrin (1110 g, 12 moles), 2-propanol (660 g, 11 moles) and water (171 g, 9.5 moles). The solution was heated to 40 °C whereupon a 20 % m aqueous sodium hydroxide solution (440 g, 2.2 moles NaOH) was added with stirring over a period of one hour. During the addition, the temperature was gradually increased to 70 - 71 °C. After a post reaction of 45 minutes at 70 - 71 °C the organic phase was separated from the aqueous brine and washed consecutively with 350 ml each of a 3 % m $NaH_2PO_4$ solution and water. Subsequently the volatiles were removed in vacuo (100 °C, 20 mbar) and the residue (320 g) was dissolved in methyl isobutyl ketone (900 g) and mixed with a 5 % m aqueous NaOH solution (400 g) at 90 °C for one hour. The organic phase was separated off, washed with 350 ml each of a 3 % m aqueous $NaH_2PO_4$ solution and water, which was followed by stripping the solvent in vacuo (140 °C, 10 mbar) during 3 hours. The final product was a pale yellow, slightly tacky epoxy resin having an epoxy group concentration: 5440 mmol/kg, saponifiable chlorine content: 90 ppm.

Examples V - VIII

Laminate preparation

Heat-hardenable compositions based on stoichiometric blends (i.e blends having an epoxy/phenolic hydroxyl equivalent ratio 1) of at least a polyglycidyl ether and at least a tetraphenylol pentane as prepared following examples I to IV were employed to prepare laminates. To this end said compounds were dissolved in methyl ethyl ketone in amounts as indicated in Table 1 hereinafter, which table also gives the amounts of ethyl triphenyl phosphonium iodide (ETPPI) catalyst which was added to each of the solutions obtained. The reactivity of said blends was determined by measuring the time to gelation at 180 °C of said solutions. The laminates were prepared by impregnating pieces of glass fibre fabric with the solutions and after drying at 20 °C for 40 minutes the impregnated pieces of fabric were cured at 180 °C for 1 hour followed by 30 minutes at 215 °C. The Tg of the laminates thus prepared was determined by Differential Scanning Calorimetry. Gelation times and Tg values are shown given in Table 1.

Comparative Experiment

Following the procedure as in examples V -VIII, a laminate based on a stoichiometric blend of a polyglycidyl ether as prepared following example III and a commercial novolac resin (ALNOVOL SPN 981 ex HOECHST) was also prepared and evaluated. The laminate composition the gelation time and the Tg value have been included in Table 1.

TABLE I

| Example | V | VI | VII | VIII | Comparative experiment |
|---|---|---|---|---|---|
| Polyglycidyl ether, g | 100.0 | 13.5 | 110.0 | - | - |
| Brominated Polyglycidyl ether, g | - | 100.0 | - | 100.0 | 100.0 |
| Tetraphenylol pentane, g | 60.5 | 48.1 | 6.0 | - | - |
| Brominated tetraphenylol pentane, g | - | - | 104.0 | 69.3 | - |
| NOVOLAC, g | - | - | - | - | 37.6 |
| ETPPI, g | 0.02 | 0.02 | 0.02 | 0.02 | 0.02 |
| Methyl ethyl ketone, g | 161 | 162 | 220 | 170 | 138 |
| Br-content, % m | - | 20.2 | 20.0 | 36.6 | 23.7 |
| Gelation time, s at 180 °C | 140 | 150 | 191 | 225 | 165 |
| Tg, °C | 223 | 218 | 217 | 224 | 182 |

**Claims**

**Claims for the following Contracting States : AT, BE, CH, DE, DK, FR, GB, GR, IT, LI, LU, NL, SE**

**1.** Heat-hardenable compositions comprising mixtures of

a) at least a polyglycidyl ether of a tetraphenol having on average at least three epoxy groups per molecule, and

b) at least a tetraphenolic crosslinking agent,

in a weight ratio sufficient to provide an epoxy group to phenolic hydroxyl group equivalent ratio in the range of from 0.5 to 1.5, and wherein the tetraphenol from which the polyglycidyl ether is derived and the tetraphenolic crosslinking agent are represented by the general formula

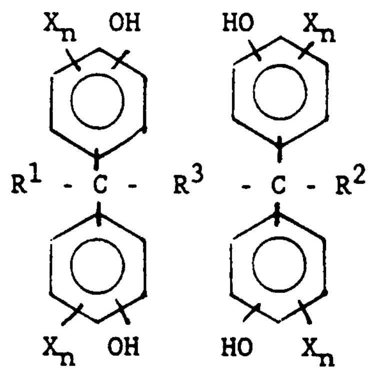

(I)

wherein $R^1$ and $R^2$ are H or a $C_{1-3}$ alkyl group, $R^3$ is a linear $C_{2-6}$ alkylene group which may carry one or more alkyl substituents, each n individually is 0,1 or 2 and X is Cl or Br.

**2.** Compositions as claimed in claim 1, wherein in general formula I $R^1$ and $R^2$ are H.

**3.** Compositions as claimed in claim 2, wherein in general formula I $R^3$ is a linear propylene group.

**4.** Compositions as claimed in any one of claims 1-3, wherein in general formula I the phenolic hydroxyl groups predominantly occupy the para position with respect to the hydrocarbon substituent.

**5.** Compositions as claimed in any one of claims 1-4, wherein in general formula I the groups X predominantly occupy an ortho position with respect to the phenolic hydroxyl groups.

**6.** Compositions as claimed in any one of claims 1-5, wherein the polyglycidyl ether has on average from 3.8 to 4.0 epoxy groups per molecule.

**7.** Compositions as claimed in any one of claims 1-6, wherein the weight ratio of polyglycidyl ether and tetraphenolic crosslinking agent is sufficient to provide an epoxy group to phenolic hydroxyl group equivalent ratio in the range of from 0.8 to 1.2.

**8.** A process for the preparation of tetraphenols having the general formula

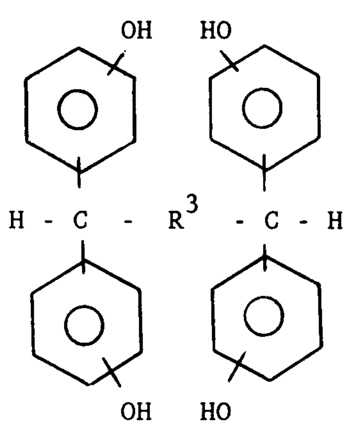

wherein $R^3$ is a linear $C_{2-6}$ alkylene group which may carry one or more alkyl substituents, by reaction of phenol and a linear $C_{4-8}$ dialdehyde in the presence of hydrochloric acid, characterized in that the

molar ratio of phenol and dialdehyde is at least 10:1 and that the hydrochloric acid is present in a concentration of at least 0.5 % mol calculated on phenol intake, and the dialdehyde is gradually added as an aqueous solution to the mixture of molten phenol and hydrochloric acid at such a rate that the temperature in the reactor does not rise above 65 °C, and wherein subsequently the volatiles are removed by distillation at elevated temperature and subatmospheric pressure.

**9.** Process for the preparation of chlorinated or brominated tetraphenols by the process as claimed in claim 8 followed by chlorination or bromination.

**Claims for the following Contracting State : ES**

**1.** A process for the preparation of heat-hardenable compositions by blending
a) at least a polyglycidyl ether of a tetraphenol having on average at least three epoxy groups per molecule, and
b) at least a tetraphenolic crosslinking agent,
in a weight ratio sufficient to provide an epoxy group to phenolic hydroxyl group equivalent ratio in the range of from 0.5 to 1.5, and wherein the tetraphenol from which the polyglycidyl ether is derived and the tetraphenolic crosslinking agent are represented by the general formula

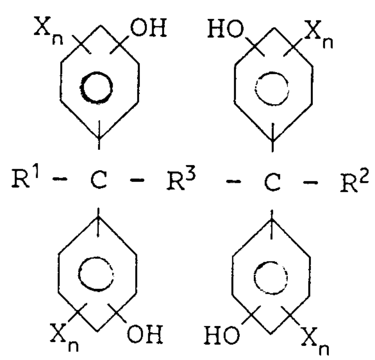

(I)

wherein $R^1$ and $R^2$ are H or a $C_{1-3}$ alkyl group, $R^3$ is a linear $C_{2-6}$ alkylene group which may carry one or more alkyl substituents, each n individually is 0,1 or 2 and X is Cl or Br.

**2.** A process as claimed in claim 1, wherein in general formula I $R^1$ and $R^2$ are H.

**3.** A process as claimed in claim 2, wherein in general formula I $R^3$ is a linear propylene group.

**4.** A process as claimed in any one of claims 1-3, wherein in general formula I the phenolic hydroxyl groups predominantly occupy the para position with respect to the hydrocarbon substituent.

**5.** A process as claimed in any one of claims 1-4, wherein in general formula I the groups X predominantly occupy an ortho position with respect to the phenolic hydroxyl groups.

**6.** A process as claimed in any one of claims 1-5, wherein the polyglycidyl ether has on average from 3.8 to 4.0 epoxy groups per molecule.

**7.** A process as claimed in any one of claims 1-6, wherein the weight ratio of polyglycidyl ether and tetraphenolic crosslinking agent is sufficient to provide an epoxy group to phenolic hydroxyl group equivalent ratio in the range of from 0.8 to 1.2.

**8.** A process for the preparation of tetraphenols having the general formula

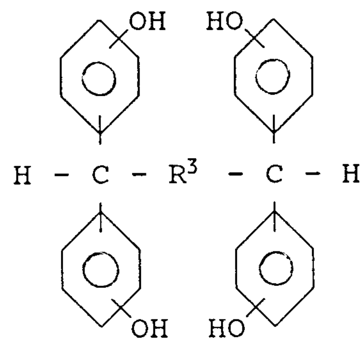

(I)

wherein $R^3$ is a linear $C_{2-6}$ alkylene group which may carry one or more alkyl substituents, by reaction of phenol and a linear $C_{4-8}$ dialdehyde in the presence of hydrochloric acid, characterized in that the molar ratio of phenol and dialdehyde is at least 10:1 and that the hydrochloric acid is present in a concentration of at least 0.5 % mol calculated on phenol intake, and the dialdehyde is gradually added as an aqueous solution to the mixture of molten phenol and hydrochloric acid at such a rate that the temperature in the reactor does not rise above 65 °C, and wherein subsequently the volatiles are removed by distillation at elevated temperature and subatmospheric pressure.

**9.** Process for the preparation of chlorinated or brominated tetraphenols by the process as claimed in claim 8 followed by chlorination or bromination.

**Patentansprüche**
**Patentansprüche für folgende Vertragsstaaten : AT, BE, CH, DE, DK, FR, GB, GR, IT, LI, LU, NL, SE**

**1.** Hitzehärtbare Zusammensetzungen, umfassend Gemische von
a) mindestens einem Polyglycidylether eines Tetraphenols mit durchschnittlich mindestens drei Epoxidgruppen pro Molekül, und
b) mindestens einem tetraphenolischen Vernetzungsmittel
in einem Gewichtsverhältnis, das ausreichend ist, um ein Äquivalenzverhältnis von Epoxidgruppen zu phenolischen Hydroxylgruppen im Bereich von 0,5 bis 1,5 zu verwirklichen, und in denen das Tetraphenol, von dem der Polyglycidylether abgeleitet ist, und das tetraphenolische Vernetzungsmittel durch die allgemeine Formel

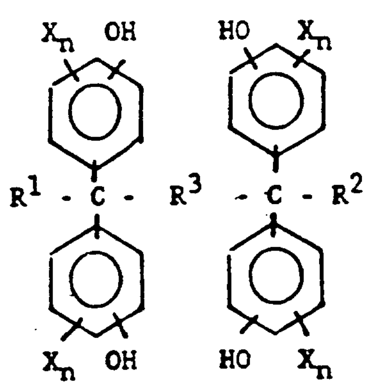

(I)

dargestellt sind, in der $R^1$ und $R^2$ H oder eine $C_{1-3}$ Alkylgruppe sind, $R^3$ eine lineare $C_{2-6}$ Alkylengruppe ist, die einen oder mehrere Alkylsubstituenten tragen kann, jedes n einzeln 0,1 oder 2 ist und X Cl oder Br bedeutet.

**2.** Zusammensetzungen wie in Anspruch 1 beansprucht, wobei in der allgemeinen Formel I $R^1$ und $R^2$ H sind.

3. Zusammensetzungen wie in Anspruch 2 beansprucht, wobei in der allgemeinen Formel I $R^3$ eine lineare Propylengruppe ist.

4. Zusammensetzungen wie in irgendeinem der Ansprüche 1 bis 3 beansprucht, wobei in der allgemeinen Formel I die phenolischen Hydroxylgruppen vorherrschend die para-Position hinsichtlich des Kohlenwasserstoff-Substituenten einnehmen.

5. Zusammensetzungen wie in irgendeinem der Ansprüche 1 bis 4 beansprucht, wobei in der allgemeinen Formel I die Gruppen X vorherrschend eine ortho-Position hinsichtlich der phenolischen Hydroxylgruppen einnehmen.

6. Zusammensetzungen wie in irgendeinem der Ansprüche 1 bis 5 beansprucht, wobei der Polyglycidylether durchschnittlich von 3,8 bis 4,0 Epoxidgruppen pro Molekül aufweist.

7. Zusammensetzungen wie in irgendeinem der Ansprüche 1 bis 6 beansprucht, wobei das Gewichtsverhältnis des Polyglycidylethers zum tetraphenolischen Vernetzungsmittel ausreichend ist, um ein Äquivalenzverhältnis der Epoxidgruppen zu den phenolischen Hydroxylgruppen im Bereich von 0,8 bis 1,2 zu verwirklichen.

8. Ein Verfahren zur Herstellung von Tetraphenolen mit der allgemeinen Formel,

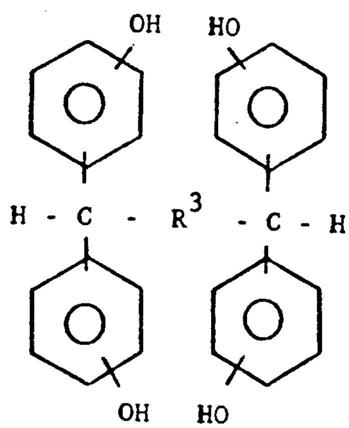

in der $R^3$ eine lineare $C_{2-6}$ Alkylengruppe ist, die einen oder mehrere Alkylsubstituenten tragen kann, durch Reaktion von Phenol und einem linearen $C_{4-8}$ Dialdehyd in Gegenwart von Salzsäure, dadurch gekennzeichnet, daß das molare Verhältnis von Phenol zu Dialdehyd mindestens 10:1 beträgt, und daß die Salzsäure in einer Konzentration von mindestens 0,5 Mol% vorliegt, gerechnet auf das Einsatzmaterial Phenol, und daß der Dialdehyd allmählich als eine wässrige Lösung zu dem Gemisch von geschmolzenem Phenol und Salzsäure in solchem Maße zugespeist wird, daß die Temperatur im Reaktor nicht über 65 °C steigt, wobei anschließend die flüchtigen Stoffe durch Destillation bei erhöhter Temperatur und unteratmosphärischem Druck abgetrennt werden.

9. Verfahren zur Herstellung von chlorierten oder bromierten Tetraphenolen durch das Verfahren, wie in Anspruch 8 beansprucht, gefolgt von Chlorierung oder Bromierung.

**Patentansprüche für folgenden Vertragsstaat : ES**

1. Ein Verfahren zur Herstellung von hitzehärtbaren Zusammensetzungen durch Mischen von
   a) mindestens einem Polyglycidylether eines Tetraphenols mit durchschnittlich mindestens drei Epoxidgruppen pro Molekül und
   b) mindestens einem tetraphenolischen Vernetzungsmittel

   in einem Gewichtsverhältnis, das ausreichend ist, um ein Äquivalenzverhältnis von Epoxidgruppen zu phenolischen Hydroxylgruppen im Bereich von 0,5 bis 1,5 zu verwirklichen, und in denen das Tetraphenol, von dem der Polyglycidylether abgeleitet ist, und das tetraphenolische Vernetzungsmittel durch die allgemeine Formel

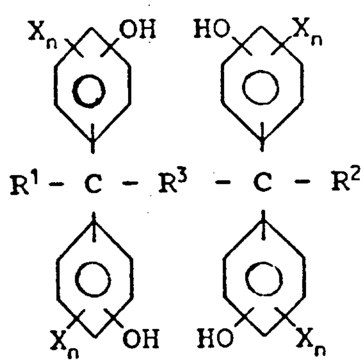

(I)

dargestellt sind, in der $R^1$ und $R^2$ H oder eine $C_{1-3}$ Alkylgruppe sind, $R^3$ eine lineare $C_{2-6}$ Alkylengruppe ist, die einen oder mehrere Alkylsubstituenten tragen kann, jedes n einzeln 0,1 oder 2 ist und X Cl oder Br bedeutet.

2. Ein Verfahren wie in Anspruch 1 beansprucht, in dem in der allgemeinen Formel I $R^1$ und $R^2$ H sind.

3. Ein Verfahren wie in Anspruch 2 beansprucht, in dem in der allgemeinen Formel I $R^3$ eine lineare Propylengruppe ist.

4. Ein Verfahren wie in irgendeinem der Ansprüche 1 bis 3 beansprucht, in dem in der allgemeinen Formel I die phenolischen Hydroxylgruppen vorherrschend die para-Position hinsichtlich des Kohlenwasserstoff-Substituenten einnehmen.

5. Ein Verfahren wie in irgendeinem der Ansprüche 1 bis 4 beansprucht, in dem in der allgemeinen Formel I die Gruppen X vorherrschend eine ortho-Position hinsichtlich der phenolischen Hydroxylgruppen einnehmen.

6. Ein Verfahren wie in irgendeinem der Ansprüche 1 bis 5 beansprucht, in dem der Polyglycidylether durchschnittlich von 3,8 bis 4,0 Epoxidgruppen pro Molekül aufweist.

7. Ein Verfahren wie in irgendeinem der Ansprüche 1 bis 6 beansprucht, in dem das Gewichtsverhältnis des Polyglycidylethers zum tetraphenolischen Vernetzungsmittel ausreichend ist, um ein Äquivalenzverhältnis der Epoxidgruppen zu den phenolischen Hydroxylgruppen im Bereich von 0,8 bis 1,2 zu verwirklichen.

8. Ein Verfahren zur Herstellung von Tetraphenolen mit der allgemeinen Formel,

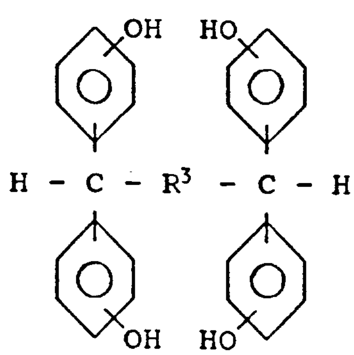

(I)

in der $R^3$ eine lineare $C_{2-6}$ Alkylengruppe ist, die einen oder mehrere Alkylsubstituenten tragen kann, durch Reaktion von Phenol und einem linearen $C_{4-8}$ Dialdehyd in Gegenwart von Salzsäure, dadurch gekennzeichnet, daß das molare Verhältnis von Phenol zu Dialdehyd mindestens 10:1 beträgt und daß die Salzsäure in einer Konzentration von mindestens 0,5 Mol%, gerechnet auf das Einsatzmaterial Phenol, vorliegt, und daß der Dialdehyd allmählich als eine wässrige Lösung zu dem Gemisch aus

geschmolzenem Phenol und Salzsäure in solch einem Maße zugespeist wird, daß die Temperatur im Reaktor nicht über 65 °C steigt, und in dem anschließend die flüchtigen Stoffe durch Destillation bei erhöhter Temperatur und subatmosphärischem Druck abgetrennt werden.

**9.** Verfahren zur Herstellung von chlorierten oder bromierten Tetraphenolen durch das Verfahren, wie in Anspruch 8 beansprucht, gefolgt von Chlorierung oder Bromierung.

**Revendications**

**Revendications pour les Etats contractants suivants : AT, BE, CH, DE, DK, FR, GB, GR, IT, LI, LU, NL, SE**

**1.** Compositions thermodurcissables comprenant des mélanges

a) d'au moins un éther polyglycidylique d'un tétraphénol ayant en moyenne au moins trois groupes époxy par molécule, et

b) d'au moins un agent de réticulation tétraphénolique, en un rapport pondéral suffisant pour fournir un rapport d'équivalents de groupe époxy au groupe hydroxyle phénolique dans la gamme de 0,5 à 1,5, et dans lequel le tétraphénol à partir duquel l'éther polyglycidylique est dérivé et l'agent de réticulation tétraphénolique sont représentés par la formule générale:

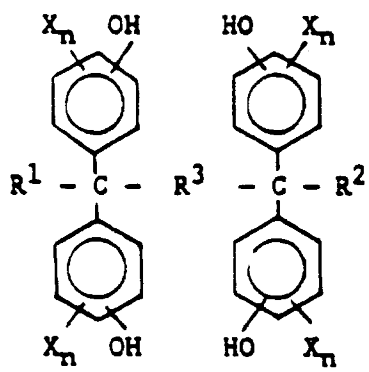

(I)

dans laquelle $R^1$ et $R^2$ sont des atomes d'hydrogène ou un groupe alkyle en $C_1$ à $C_3$, $R^3$ est un groupe alkylène linéaire en $C_2$ à $C_6$ qui peut porter un ou plusieurs substituants alkyle, chaque n vaut individuellement 0,1 ou 2 et X représente un atome de Cl ou Br.

**2.** Compositions selon la revendication 1, dans lesquelles, dans la formule générale (I) $R^1$ et $R^2$ sont des atomes d'hydrogène.

**3.** Compositions selon la revendication 2, dans lesquelles dans la formule générale (I) $R^3$ est un groupe propylène linéaire.

**4.** Compositions selon l'une quelconque des revendications 1 à 3, dans lesquelles dans la formule générale I les groupes hydroxyle phénolique occupent de façon prédominante la position para par rapport au substituant hydrocarboné.

**5.** Compositions selon l'une quelconque des revendications 1 à 4, dans lesquelles dans la formule générale I le groupe X occupe de façon prédominante une position ortho par rapport aux groupes hydroxyle phénolique.

**6.** Compositions selon l'une quelconque des revendications 1 à 5, dans lesquelles l'éther polyglycidylique a en moyenne 3,8 à 4,0 groupes époxy par molécule.

**7.** Compositions selon l'une quelconque des revendications 1 à 6, dans lesquelles le rapport pondéral d'éther polyglycidylique et de l'agent de réticulation tétraphénolique est suffisant pour fournir un groupe époxy à un rapport d'équivalents de groupe époxy au groupe hydroxyle phénolique dans la gamme de 0,8 à 1,2.

8. Procédé pour la préparation de tétraphénols ayant la formule générale:

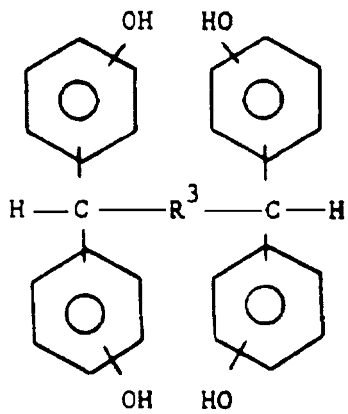

dans laquelle $R^3$ est un groupe alkylène linéaire en $C_2$ à $C_6$ qui peut porter un ou plusieurs substituants alkyle, par réaction du phénol et d'un dialdéhyde linéaire en $C_4$ à $C_8$ en présence d'acide chlorhydrique, caractérisé en ce que le rapport molaire de phénol et de dialdéhyde est d'au moins 10:1 et en ce que l'acide chlorhydrique est présent en une concentration d'au moins 0,5% en moles calculée par rapport à l'introduction de phénol, et que le dialdéhyde est ajouté graduellement sous forme d'une solution aqueuse au mélange de phénol fondu et d'acide chlorhydrique à un débit tel que la température dans le réacteur ne s'élève pas au-dessus de 65 °C, et dans lequel on élimine ultérieurement les produits volatils par distillation à température élevée et pression inférieure à la pression atmosphérique.

9. Procédé pour la préparation de tétraphénols chlorés ou bromés par le procédé selon la revendication 8 suivi de chloration ou de bromation.

**Revendications pour l'Etat contractant suivant : ES**

1. Procédé pour la préparation de compositions thermodurcissables comprenant des mélanges
   a) d'au moins un éther polyglycidylique d'un tétraphénol ayant en moyenne au moins trois groupes époxy par molécule, et
   b) d'au moins un agent de réticulation tétraphénolique, en un rapport pondéral suffisant pour fournir un rapport d'équivalents de groupe époxy au groupe hydroxyle phénolique dans la gamme de 0,5 à 1,5, et dans lequel le tétraphénol à partir duquel l'éther polyglycidylique est dérivé et l'agent de réticulation tétraphénolique sont représentés par la formule générale:

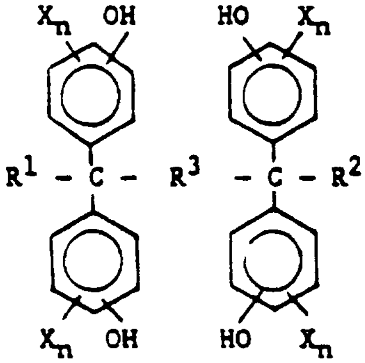

(I)

dans laquelle $R^1$ et $R^2$ sont des atomes d'hydrogène ou un groupe alkyle en $C_1$ à $C_3$, $R^3$ est un groupe alkylène linéaire en $C_2$ à $C_6$ qui peut porter un ou plusieurs substituants alkyle, chaque n vaut individuellement 0,1 ou 2 et X représente un atome de Cl ou Br.

**2.** Procédé selon la revendication 1, dans lequel dans la formule générale (I) $R^1$ et $R^2$ sont des atomes d'hydrogène.

**3.** Procédé selon la revendication 2, dans lequel dans la formule générale (I) $R^3$ est un groupe propylène linéaire.

**4.** Procédé selon l'une quelconque des revendications 1 à 3, dans lequel dans la formule générale I les groupes hydroxyle phénolique occupent de façon prédominante la position para par rapport au substituant hydrocarboné.

**5.** Procédé selon l'une quelconque des revendications 1 à 4, dans lequel dans la formule générale I le groupe X occupe de façon prédominante une position ortho par rapport aux groupes hydroxyle phénolique.

**6.** Procédé selon l'une quelconque des revendications 1 à 5, dans lequel l'éther polyglycidylique a en moyenne 3,8 à 4,0 groupes époxy par molécule.

**7.** Procédé selon l'une quelconque des revendications 1 à 6, dans lequel le rapport pondéral d'éther polyglycidylique et de l'agent de réticulation tétraphénolique est suffisant pour fournir un groupe époxy à un rapport d'équivalents de groupe époxy au groupe hydroxyle phénolique dans la gamme de 0,8 à 1,2.

**8.** Procédé pour la préparation de tétraphénols ayant la formule générale:

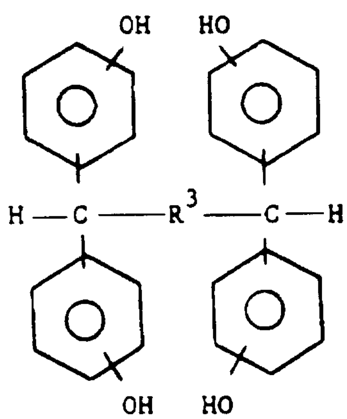

dans laquelle $R^3$ est un groupe alkylène linéaire en $C_2$ à $C_6$ qui peut porter un ou plusieurs substituants alkyle, par réaction du phénol et d'un dialdéhyde linéaire en $C_4$ à $C_8$ en présence d'acide chlorhydrique, caractérisé en ce que le rapport molaire de phénol et de dialdéhyde est d'au moins 10:1 et en ce que l'acide chlorhydrique est présent en une concentration d'au moins 0,5% en moles calculée par rapport à l'introduction de phénol, et que le dialdéhyde est ajouté graduellement sous forme d'une solution aqueuse au mélange de phénol fondu et d'acide chlorhydrique à un débit tel que la température dans le réacteur ne s'élève pas au-dessus de 65 °C, et dans lequel on élimine ultérieurement les produits volatils par distillation à température élevée et pression inférieure à la pression atmosphérique.

**9.** Procédé pour la préparation de tétraphénols chlorés ou bromés par le procédé selon la revendication 8 suivi de chloration ou de bromation.